# EUROPEAN PATENT APPLICATION

(11) **EP 1 525 852 A1**
(43) Date of publication of application: **27.04.2005**
(21) Application number: 03760936.9
(22) Date of filing: 20.06.2003
(51) Int. Cl.: A61B 17/28, A61M 25/00, A61M 31/00

(54) **CATHETER**

(30) Priority: 21.06.2002 JP 2002181726
(71) Applicant: Kabushiki Kaisha Vayu, Nagoya-shi, Aichi 461-0023 (JP)
(72) Inventor: HORIBA, Mitsuru, Yagoto-omoteyama-jutaku 2-205, Nagoya-shi, Aichi468-0069 (JP); TSUTSUI, Nobumasa, c/o K.K. Vayu, Nagoya-shi, Aichi 461-0023 (JP); TSUTSUI, Yasuhiro, Nagoya-shi, Aichi 461-0023 (JP)
(74) Representative: Intès, Didier Gérard André
(86) International application number: PCT/JP2003/007899
(87) International publication number: WO 2004/000142

(57) **Abstract**

A catheter which allows injection into a target region without imposing large burden on a medical patient can be provided. A catheter 1 is used by being inserted from outside of a body into a blood vessel and a distal end thereof being reached inside of a heart, that is a target region, while a proximate end thereof remaining outside of the body. The catheter 1 is configured in a way so that an injection mechanism 5 conducts injection into myocardium from inside of a heart while a forceps mechanism 3 holds myocardium inside of the heart. In the forceps mechanism 3, a grasping portion 13 provided at the distal end of the catheter 1 opens and closes in conjunction with manipulation at a first handling portion 11 provided at the proximate end of the catheter 1. In the injection mechanism 5, an injection needle 43 provided at the distal end of the catheter 1 is protruded from the distal end of the catheter 1 in conjunction with manipulation at a second handling portion 41 provided at the proximate end of the catheter 1.

## Description

### TECHNICAL FIELD

The present invention relates to a catheter.

### BACKGROUND ART

In recent years, vascularization wherein a blood vessel is vascularized in an ischemic heart by injecting various kinds of injectant including a vascularization factor (such as protein, genes etc.) has been conducted. By this treatment method, this treatment can be applied to some cases for those medical patients to whom it is difficult to conduct Percutaneous Transluminal Coronary Angioplastry (PTCA) or Coronary Artery Bypass Grafting (CABG), and this treatment method has been drawing attention as a new treatment method.

Meanwhile, in case myocardial cells become necrotized from myocardial infarction or myocarditis, remaining myocardium increasingly enlarges and compensates for reduced cardiac function. However, if this compensation fails, myocardium is caused. Organ regeneration, wherein lost myocardial cells are regenerated by injecting embryo myocardial cells, skeletal muscle cells, smooth muscle cells, marrow cells or ES cells into myocardium, has been considered. Today, certain degree of effect in animal experiments, mainly in cell transplants, has been reported. It seems regeneration of myocardium might be possible in the future by gene introduction. Moreover, treatment which can be adaptable for dilated cardiomyopathy and minimally invasive to patients with no other treatment than heart transplants is expected. Additionally, it seems this kind of organ regeneration is going to be widely considered for other diseases, as well as for heart diseases.

Furthermore, as treatment for bradyarrhythmia, instead of implanting a pacemaker, generation of spontaneous pulsation of myocardium (a biological pacemaker) has been attempted. For this treatment, injection of various kinds of injectant (for example, cell transplants, gene introduction, or administration of some kind of hormone preparation) into ventricular myocardium has been considered.

However, when the various kinds of treatment described above are conducted, injection into an organ (such as myocardium) needs to be conducted, accordingly surgical thoracotomy and direct injection into treatment region have to be conducted. In this way, invesiveness to a patient is tremendous, and this has been a problem.

The present invention is to solve above problems, and the purpose is to provide a catheter with capability of injection into a target region without imposing enormous burden on a patient.

### DISCLOSURE OF INVENTION

In order to attain the above object, a catheter of the present invention is a catheter used by being inserted into a coelom from outside of a body, and a distal end thereof being reached to a target region while a proximate end thereof being remained outside of the body. The catheter comprises: a forceps mechanism having a fist handling portion provided at the proximate end and a grasping portion provided at the distal end, wherein the grasping portion opens and closes in conjunction with manipulation at the first handling portion, and wherein the grasping portion is capable of holding the target region; and an injection mechanism having a second handling portion provided at the proximate end and an injection needle provided at the distal end, wherein the injection needle is moved forward up to a position to be protruded from the distal end and is moved back up to a position to be stored inside of the distal end, and wherein the injection needle is capable of puncturing the target region to inject injectant into the target region.

In this catheter, specific configuration of detail parts of the forceps mechanism can be any configuration, as long as the grasping portion provided at the distal end opens and closes in conjunction with manipulation at the first handling portion provided at the proximate end. For example, a configuration, wherein one wire is lead through in center of a catheter and one end of the wire is connected to the grasping portion, the grasping portion opens when pushed by the wire, and closes when pulled by the wire, in association with reciprocation of the wire along an axial direction created by manipulation of the first handling portion at the proximate end, can be possible. Alternatively, a power transmission mechanism, wherein turning of a similar wire is converted into opening/closing movement of the grasping portion, can be configured.

Specific configuration of detail parts of the injection mechanism can be any configuration, as long as the injection needle provided at the distal end moves forward up to a position to be protruded from the distal end and moves back up to a position to be stored inside of the distal end in conjunction with manipulation at the second handling portion provided at the proximate end, and the injection needle punctures a target region to allow injectant injected into the target region. For example, following configuration can be possible: one tube is lead through in center of a catheter and one end of the tube is connected with the injection needle. When the tube is reciprocated along with an axial direction by manipulating the second handling portion provided at the proximate end, the injection needle is reciprocated together with the tube. The injection needle is protruded from the distal end while moving forward, and is stored inside of the distal end while moving back. In this case, a lumen of the tube can be used as an injectant supply path to supply injectant to the injection needle. Alternatively, a wire can be disposed in parallel to the tube for moving the injection needle forward/backward in association with reciprocate movement of the wire, so that the tube can be used only as an injectant supply path. In case this kind of tube is provided, a syringe can be connected to the tube at the proximate end of the catheter, and injectant is injected by using the syringe. Accordingly the injectant can be supplied to the injection needle through the tube. In order to supply injectant, a tube described above does not have to be used. A supplier can be configured, for example, to have a injectant storage formed at the distal end of the catheter, and when pressure is applied to the injectant storage corresponding to manipulation at the proximate end of the catheter, injectant is released from the injectant storage and supplied to the injection needle.

As for injectant, any injectant can be used, as long as the injectant can be injected through the injection needle and suitable for a treatment target. However, since some types of medicine, genes, protein and cells are considered for vascularization and organ regeneration, use of these kinds of injectant can be possible.

It should be noted that other than the above-described forceps mechanism and the injection mechanism, a configuration that other catheters for inserting into coelom have can be used together. For example, if a guide wire is used for inserting the catheter into coelom, a tube that provides a lumen to lead through the guide wire can be disposed in parallel to the above-described forceps mechanism and the injection mechanism. Alternatively, a lumen for measuring blood pressure, or a lumen for securing bloodstream, that derives blood introduced from one end thereof to another end, can be provided. Moreover, a reinforcement member or covering member in order to improve strength and slipperiness of the catheter can be also voluntarily used within a scope not to prevent main function of the present catheter in consideration of place where this catheter is used.

In usage of a catheter configured as above, first the catheter is inserted into coelom from outside of a body, and while the proximate end is remained outside of the body, the distal end is reached to a target region. As for methods to insert the catheter into coelom and to reach the distal end to a target region, same methods can be voluntarily used as methods used for various catheters for inserting coelom. When the distal end is pushed toward the target region, the injection needle of the injection mechanism should be moved back, in advance, up to a position to be stored inside of the distal end of the catheter. Thereby, puncture of the injection needle into an unexpected part can be prevented.

Once the distal end of the catheter is reached to the target region, the target region is held with the grasping portion of the forceps mechanism. Thereby, even the target region moves, displacement of the distal end of the catheter in relation with the target region can be prevented. When the relational position of the distal end of the catheter and the target region is fixed by the forceps mechanism, the injection needle of the injection mechanism is moved forward to puncture the target region, and injection into the target region is conducted. At this time, if injectant has high consistency, or if relatively large amount of injectant needs to be injected into one spot, force toward a direction to push back the injection needle is applied to the injection needle corresponding to injection of injectant. However, in this catheter, since the grasping portion of the forceps mechanism is holding the target region, the injection needle does not get pushed back or pulled out, and high stability during injection can be maintained.

After conducting injection with above-described procedure, the catheter is removed in same manner as various catheters, that are inserted into coelom, are removed.

With a catheter described above, injection into a target region can be conducted by using the catheter. Moreover, appropriate injection can be conducted while preventing displacement of the distal end of the catheter in relation with the target region, especially even if the target region moves.

For more specific example, this catheter can preferably conduct injection into myocardium. That is, displacement of the distal end of the catheter in relation with inner surface of a heart can be prevented by inserting the catheter into a blood vessel, reaching the distal end to inside of the heart, and holding the inner surface of the heart with the grasping portion of the above-described forceps mechanism. Injection can be conducted into myocardium from inside of the heart with the injection mechanism. Specially, since the grasping portion of the forceps mechanism can hold inner surface of a heart, injection into myocardium can be easily and appropriately conducted without the distal end of the catheter displaced in relation with inner surface of a heart, even the heart pulsates.

Therefore, for conducting vascularization, organ regeneration, or treatment with a biological pacemaker, injection into a treatment region such as myocardium can be conducted without surgical thoracotomy, and burden imposed on a patient can be reduced.

The catheter described above becomes more preferable with following configuration.

The forceps mechanism can be configured to bias the grasping portion toward a direction to close the grasping portion with force of a spring.

In a catheter configured as above, once the grasping portion of the forceps mechanism is opened, the grasping portion can be closed with the force of the spring. Thus, it is not necessary to apply external force to close the grasping portion, and the grasping portion can be easily closed. Moreover, since closed state of the grasping portion can be maintained with the force of the spring, it is easier to keep the grasping portion closed, compared to a configuration wherein external force needs to be continuously applied in order to keep the closed state.

Furthermore, the forceps mechanism may be provided with a lock device that forbids the grasping portion opening/closing.

In a catheter configured as above, the closed state of the grasping portion can be maintained by the lock device of the forceps mechanism. Thus, when the grasping portion holds a target region, the state of the grasping portion can be more easily maintained, compared to a configuration wherein external force needs to be continuously applied in order to keep the grasping portion closed.

Still furthermore, the injection mechanism may be configured to bias the injection needle toward a direction to move back the injection needle with the force of a spring.

In a catheter configured as above, after being moved forward, the injection needle can be moved back with the force of the spring and stored inside the catheter. Thus, it is not necessary to apply external force to move back the injection needle, and the injection needle can be easily moved back. Moreover, since state, wherein the injection needle is moved back, can be maintained with the force of the spring, the injection needle can be maintained more easily in the retreated state, compared to a configuration wherein external force needs to be continuously applied in order to maintain the retreated state.

Still furthermore, the injection mechanism may be provided with a lock device that forbids the injection needle moving back.

In a catheter configured as above, state, wherein the injection needle is moved forward, can be maintained with the lock device of the injection mechanism. Thus, it is easier to maintain the advanced state of the injection needle, compared to a configuration wherein external force needs to be continuously applied in order to maintain the advanced state, and the injection needle does not get pulled out of a target region by being accidentally moved back. Moreover, since the retreated state of the injection needle can be maintained by the lock device of the injection mechanism, it is easier to maintain the retreated state of the injection needle, compared to a configuration wherein external force needs to be continuously applied in order to keep the retreated state. Thus, accidental puncture of the injection needle into an unexpected body part can be prevented.

In addition to above-described configurations, a configuration, wherein a third handling portion is provided at the proximate end, and the distal end is curved in conjunction with manipulation at the third handling portion, can be possible.

In a catheter configured as above, since a position of the distal end can be adjusted by curving the distal end, and a puncture position of the injection needle can be more accurately controlled.

Moreover, for position adjustment of the distal end, a position of the distal end of the catheter of the present invention can be adjusted by introducing a guide catheter having a configuration capable of controlling direction of a leading end into coelom, introducing the catheter of the present invention into a lumen of this guide catheter, and manipulating the guide catheter.

### BRIEF DESCRIPTION OF DRAWINGS

Figs. 1A to 1C are schematic diagrams of a catheter described in an embodiment of the present invention;

Figs. 2A and 2B are sectional views showing details of a forceps mechanism; and

Figs. 3A and 3B are sectional views showing details of an injection mechanism.

### BEST MODE FOR CARRYING OUT THE INVENTION

Preferred embodiments of the present invention will now be described with some examples.

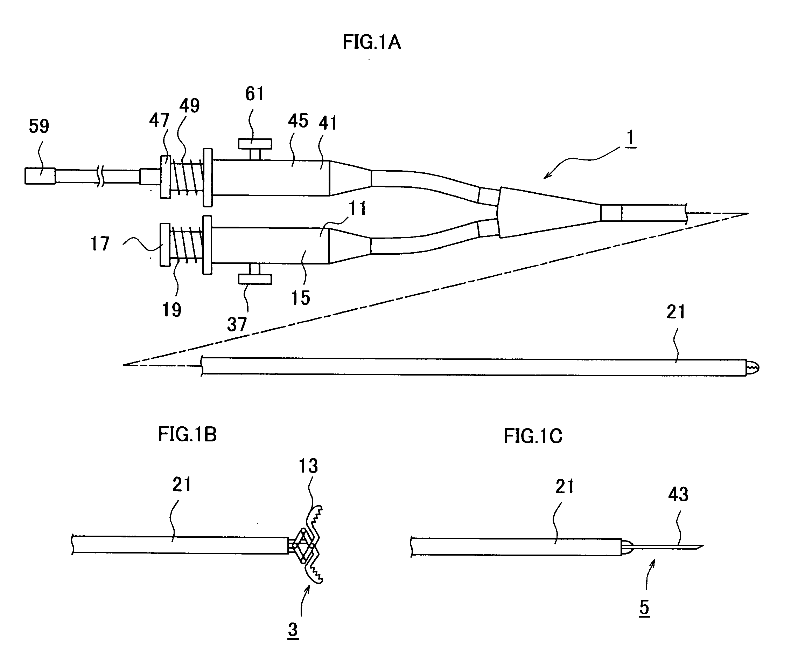

Figs. 1A to 1C are schematic diagrams of a catheter 1 shown as an embodiment of the present invention.

This catheter 1 is used by being inserted into a blood vessel from outside of a body, and a distal end thereof being reached to a target region, i.e. inside of a heart, while a proximal end thereof being remained outside of the body. The catheter 1 is configured to conduct injection into myocardium with an injection mechanism 5, while holding the myocardium with a forceps mechanism 3 from inside of the heart.

In the forceps mechanism 3, a grasping portion 13, provided at the distal end of the catheter 1, opens and closes in conjunction with manipulation with a first handling portion 11 provided at the proximate end of the catheter 1.

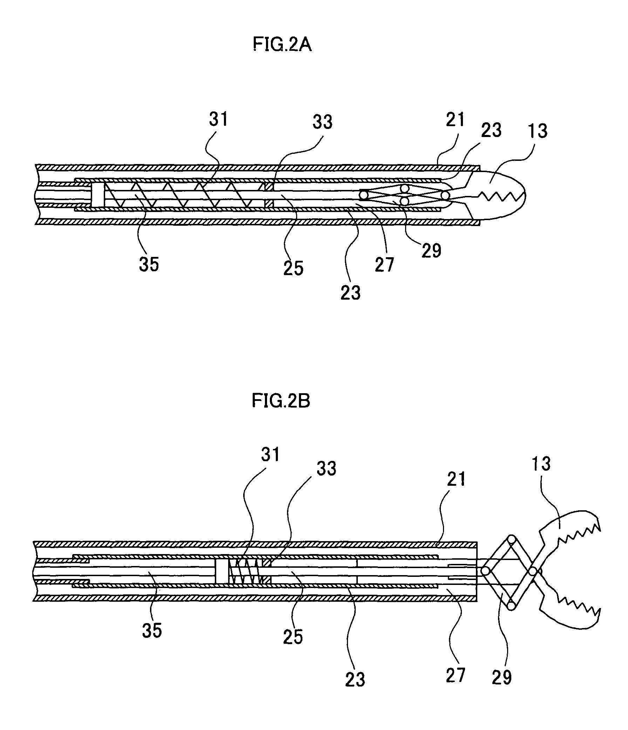

More specifically, the first handling portion 11 comprises a cylinder 15, a piston 17, and a spring 19. When external force is applied, the piston 17 can be pushed into the cylinder 15. When external force is relieved, the piston 17 is returned to an original position by force of the spring 19. In a vicinity of the grasping portion 13, as shown in Figs. 2A and 2B, an outer tube 21, a first inner tube 23, a rod 25, a slider 27, a link 29, a spring 31, and stopper 33 are provided. One end of a wire 35 led through inside of the catheter 1 is connected to the above-mentioned rod 25, and another end of the wire 35 is connected to the above-mentioned piston 17. On a lateral side of the cylinder 15, a screw 37 is provided as a lock device of the forceps mechanism. By tightening this screw 37, the piston 17 can be fixed in the cylinder 15.

In the forceps mechanism 3 with the configuration described above, when the piston 17 is pushed into the cylinder 15, the wire 35 is correspondingly pushed out toward a direction of the distal end, the rod 25 is pushed by the wire 35, and the rod 25 compresses the spring 31 while pressing the link 29. By restriction of the first inner tube 23, the link 29 cannot be laterally expanded. Until going outside of the first inner tube 23, the link 29 transfers expansion force thereof to the slider 27 without being expanded, and the slider 27 slides toward a direction of an leading end of the forceps mechanism 3. When the link 29 goes outside of the first inner tube 23, the slider 27 slides and the link 29 expands laterally. The slider 27 is disposed in a manner so that movement thereof is restricted after sliding for specific amount. Consequently, the movement of the slider 27 stops after some portion of the slider 27 is protruded from the outer tube 21. Subsequently, the link 29 is pushed by the rode 25 and furthermore expanded laterally, and the grasping portion 13 opens widely.

After the grasping portion 13 is opened as described above, the force applied to the piston 17 is relieved, and the piston 17 is returned to the original position by the force of the spring 19. Correspondingly, the wire 35 is pulled toward the direction of the proximate end, and at the same time, the rod 25 is pulled by the force of the spring 31. The link 29 is pulled toward the direction of the proximate end by these forces. As the laterally expanded width of the link 29 gets hung up on an end face of the outer tube 23, the width is narrowed, and the link 29 is withdrawn inside of the first inner tube 23. Concurrently, the slider 27 is withdrawn inside of the first inner tube 23. The grasping portion 13 is closed in conjunction with this movement of the link 29.

The injection mechanism 5 is configured in a manner so that an injection needle 43, provided at the distal end of the catheter 1, is protruded from the distal end of the catheter 1 in conjunction with manipulation at a second handling portion 41 provided at the proximate end of the catheter 1.

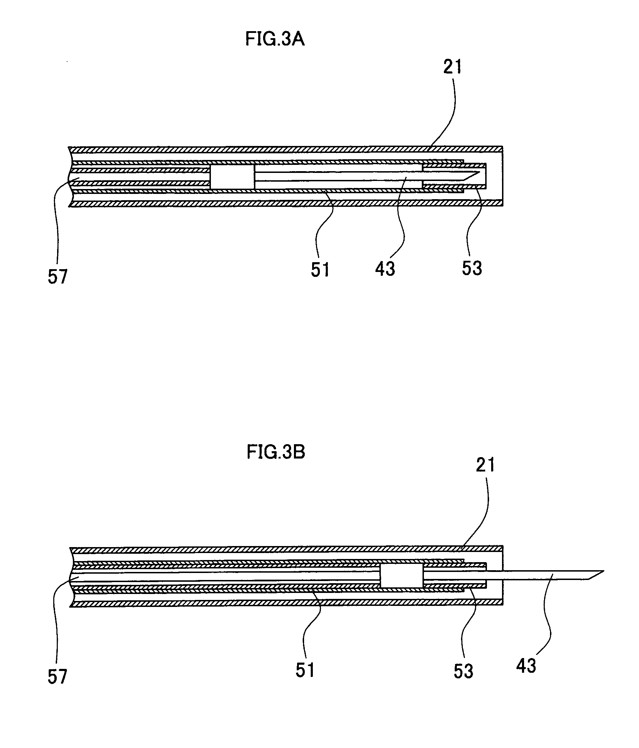

More specifically, the second handling portion 41 comprises a cylinder 45, a piston 47, and a spring 49, as well as the first handling portion 11. When external force is applied, the piston 47 can be pushed into the cylinder 45. When the external force is relieved, the piston 47 is returned to an original position thereof by force of the spring 49. Moreover, as shown in Figs. 3A and 3B, in vicinity of the injection needle 43, the above-described outer tube 21, a second inner tube 51, and a stopper 53 are provided. One end of an injectant supply tube 57 lead through inside of the catheter 1 is connected to the injection needle 43. Another end of the injectant supply tube 57 is introduced from the above-mentioned piston 47, and a syringe connecting portion 59, wherein a syringe (not shown) for injecting injectant can be connected thereto, is formed. The injectant supply tube 57 is fixed so as not to be displaced in relation with the above-described piston 47. Additionally, a screw 61 is provided on a lateral surface of the cylinder 45 as a lock device of the injection mechanism. By tightening this screw 61, the piston 47 can be fixed to the cylinder 45.

In Figs. 3A and 3B, to avoid complication in the drawings, the above-described forceps mechanism 3 is not illustrated. However, the outer tube 21 shown in Figs. 3A and 3B is the same as the outer tube 21 shown in Figs. 2A and 2B. The first inner tube 23 shown in Figs. 2A and 2B and the second inner tube 51 shown in Figs. 3A and 3B are disposed in parallel inside of the outer tube 21.

In the injection mechanism 5 with a configuration described above, when the piston 47 is pushed into the cylinder 45, the injectant supply tube 57 is correspondingly pushed out toward the direction of the distal end. The injection needle 43 is pushed by the injectant supply tube 57, and the injection needle 43 is protruded from the outer tube 21. Movement of the injection needle 43 is restricted when the injection needle 43 abuts on the stopper 53. Thus, maximum protruding amount of the injection needle 43 becomes constant.

After the injection needle is protruded and the force applied to the piston 47 is relieved, the piston 47 is returned to the original position by the force of the spring 49. Correspondingly, the injectant supply tube 57 is pulled toward the direction of the proximate end, and thereby the injection needle 43 is pulled into inside of the second inner tube 51.

The catheter 1 configured as above can be used according to following procedure.

First, the catheter 1 is inserted into a blood vessel from outside of a body. Leaving the proximate end thereof outside of the body, the distal end thereof is reached into inside of a heart. For insertion, a puncture needle is punctured and a sheath is inserted in advance, as well as for inserting a known catheter. The catheter 1 can be introduced through a lumen of the sheath. As regards a method of reaching the distal end of the catheter 1 into inside of a heart, a same method of a catheter, used by reaching a leading thereof into a heart, can be voluntarily used. For example, a method wherein a tubular guide catheter is reached into inside of a heart in advance and the catheter 1 is inserted along a lumen of the guide catheter, or a method wherein a guide wire is reached into inside of a heart in advance and the catheter 1 is inserted along the guide wire can be voluntarily used.

When the distal end of the catheter 1 is inserted into inside of a heart, the injection needle 43 provided in the injection mechanism 5 is moved back to a position to be stored inside of the distal end of the catheter 1. By tightening the screw 61, the piston 47 is locked, and the injection needle 43 does not get pushed out, even if the piston 47 is touched. Thereby, puncturing unexpected spot with the injection needle 43 can be prevented.

When the distal end of the catheter 1 reaches inside of a heart, myocardium is held with the grasping portion 13 of the forceps mechanism 3. When holding myocardium, the grasping portion 13 is opened and closed by manipulating the piston 17 at the first handling portion 11. Although, the piston 17 actively moves toward a closing direction by the force of the spring 19 and the like, the piston 17 can be manually pulled. When myocardium is held with the grasping portion 13 by the above-described manipulation, the screw 37 is tightened to lock the piston 17. Thereby, the holing of myocardium with the grasping portion 13 does not get released even if the piston 17 is touched.

Consequently, when myocardium is held with the grasping portion 13 as above, even the myocardium moves corresponding to pulsation, a position of the distal end of the catheter 1 in relation with myocardium does not get displaced.

In the above state, the injection needle 43 of the injection mechanism 5 is moved forward to puncture myocardium, and injection into myocardium is conducted. When puncturing myocardium, the screw 61 is once untightened to release the locking of the piston 47, the piston 47 is subsequently pushed into the cylinder 45, and then the screw 61 is tightened again to lock the movement of the piston 47. Thereby, fallback and slipping out of the injection needle 43 can be prevented. In this state, injection into myocardium can be conducted by using a syringe (not shown) connected to the syringe connecting portion 59, and injecting injectant. At this time, if injectant has high consistency, or if relatively large amount of injectant needs to be injected into one spot, the injection needle 43 receives force toward a direction to which the injection needle 43 is pushed back corresponding to injection of injectant. However, in this catheter 1, since the grasping portion 13 of the forceps mechanism 3 is holding myocardium, the injection needle 43 is not pushed back or slipped out. Thus, high stability during injection can be maintained.

It should be noted that in the above procedure, the step of puncture into myocardium and steps after puncture can be repeated several times in the same manner. Thereby, injection into several parts of myocardium can be easily conducted.

After conducting injection, the catheter 1 is removed in a same method as a method of various catheters which are inserted into a coelom.

With the catheter 1 described above, injection into myocardium can be conducted by using the catheter 1. Specially, even when myocardium moves corresponding to pulse of a heart, displacement of the position of the distal end of the catheter 1 in relation with myocardium can be prevented, and injection can be appropriately conducted.

Consequently, when vascularization, wherein a blood vessel is vascularized in an ischemic heart, is conducted, injection into myocardium can be conducted without surgical thoracotomy operation, and burden on a patient can be reduced.

Although, an embodiment of the present invention is described above, the present invention is not limited to a specific embodiment described above, and can be conducted in various forms other than the above embodiment.

For example, although the above embodiment does not mention, in addition to the forceps mechanism 3 and the injection mechanism 5, additional constituents provided in other catheters which are inserted into a blood vessels can be also used.

Specifically, if a guide wire is used when the catheter 1 is inserted into a blood vessel, a tube that provides a lumen to lead the guide wire can be disposed in parallel to the above-described forceps mechanism and the injection mechanism. A lumen for measuring blood pressure, or a lumen for ensuring bloodstream to derive blood introduced from one end to another end can be provided. Alternatively, reinforcement member or covering member in order to improve strength and slipperiness of the catheter can be also used voluntarily within a scope not to prevent the main function of the catheter in consideration of place where this catheter is used.

Moreover, a third handling portion can be provided at the proximate end of the catheter 1 to make a configuration so that the distal end of the catheter 1 is curved in association with manipulation at the third handling portion and a direction of the distal end can be voluntarily changed. With a catheter configured as above, a position of the distal end can be adjusted by curving the distal end. Thus, a puncture position of the injection needle 43 can be more precisely controlled.

In the above embodiment, usage of the catheter 1 in vascularization is specifically described. Usage of the above catheter 1 can be also applied to organ regeneration for cardiomyopathy, or introduction of so-called a biological pacemaker for arrhythmia. Furthermore, not only for treatment intended for heart, but a catheter adopting the configuration of the present invention can be also used for injection into other parts of a body in addition to myocardium. In that case, it goes without saying that corresponding to a target region, length and thickness of the catheter can be appropriately changed. Still furthermore, corresponding to strength or flexibility required to the catheter, various kinds of resin materials used for manufacturing this type of catheter can be used in voluntary combination.

Additionally, in the above-described embodiment, the catheter 1 is provided with the scissors-like (beak shaped) grasping portion 13 configured with two pieces of movable portions. Depending on a grasping target, a grasping portion can be configured with more than two movable portions that open and close radially. Furthermore, in the above-described embodiment, some portion of the grasping portion that comes in contact with a grasping target is serrated. However, any shape, such as a shape with several spines protruding, can be voluntarily adopted, as long as the contact portion can steadily hold a grasping target.

### INDUSTRIAL APPLICABILITY

The present invention renders a catheter that allows injection into a target region without imposing large burden to a patient. With this catheter, injection, for example, into myocardium while holding myocardium inside a heart can be conducted.

## Claims

1. A catheter used by being inserted from outside of a body into a coelom and reaching a distal end thereof to a target region while a proximate end thereof being remained outside of the body, the catheter comprising:
a forceps mechanism having a fist handling portion at the proximate end and a grasping portion at the distal end, the grasping portion being configured to open and close in conjunction with manipulation at the first handling portion, and being capable of holding the target region;
an injection mechanism having a second handling portion at the proximate end, and an injection needle at the distal end, the injection needle being configured to be moved forward up to a position to be protruded from the distal end, and to be moved back up to a position to be stored inside of the distal end, the injection mechanism being capable of puncturing the target region with the injection needle and injecting injectant into the target region.

2. The catheter as set forth in claim 1 wherein the forceps mechanism is configured to bias the grasping portion toward a direction to close the grasping portion with force of a spring.

3. The catheter as set forth in claim 1 or 2 wherein the forceps mechanism comprises a lock device that forbids the grasping portion opening and closing.

4. The catheter as set forth in any of claim 1 to 3 wherein the injection mechanism is configured to bias the injection needle toward a direction to move back the injection needle with force of a spring.

5. The catheter as set forth in any of claim 1 to 4 wherein the injection mechanism comprises a lock device that forbids the injection needle moving back.
